# EUROPEAN PATENT SPECIFICATION

(11) **EP 0 679 076 B1**
(45) Date of publication and mention of the grant of the patent: **21.07.1999**
(21) Application number: 93903432.8
(22) Date of filing: 12.01.1993
(51) Int. Cl.: A61F 13/00, A61F 15/00, A61L 15/00

(54) **INVISIBLE BANDAGE ASSEMBLY**
UNSICHTBARER VERBAND
PANSEMENT INVISIBLE

(43) Date of publication of application: 02.11.1995
(73) Proprietor: NEWMAN, Nancy M., Mill Valley, CA 94941 (US)
(72) Inventor: NEWMAN, Nancy M., Mill Valley, CA 94941 (US)
(74) Representative: Price, Nigel John King
(86) International application number: US9300263
(87) International publication number: WO9415561

(56) References cited:
- DE-U- 8 811 658
- US-A- 3 297 034
- US-A- 4 561 435
- US-A- 4 730 611
- US-A- 4 998 617
- US-A- 5 117 981
- US-A- 5 368 553

## Description

This invention is in the medical and cosmetic fields. More specifically, it concerns bandages useful for a variety of medical applications and as dermatologic cover-ups. Still, more specifically, it relates to bandages which are not readily apparent to an observer, that is, "invisible" as that term is defined herein.

There are many commercially available types of bandages of different shapes and sizes, most of which are monochromatic--of a beige color that is apparently designed to match the skin tone of certain Caucasians. Exemplary of such bandages are BAND-AID™ Sheer Bandages [Johnson & Johnson Products Inc.; New Brunswick, N.J. (U.S.A.)].

Also available commercially are a spray-on adhesive-type of bandage, for example, plastic spray-on bandages. Such spray-on bandages produce a relatively transparent cover which is generally occlusive and tends to dry and crack.

There is a need in the art for bandages which can be used to perform medical functions in a cosmetically acceptable manner and/or to provide coverage for unsightly dermatologic conditions. This invention fulfills that need and further meets other requirements that are desirable for cover-ups and wound dressings.

The "invisible bandages" of this invention are flexible and conform to the contours of the section of the body to which they are to be applied, and when medically appropriate, have a specified degree of occlusivity and/or are non-adherent to wounds. The invisible bandages of this invention are not only designed to be cosmetically acceptable and comfortable, but also can be used to promote wound healing and provide vehicles for applying a variety of medicaments. Rather than being monochromatic, the invisible bandages of this invention are designed to match the skin of the individual to whom the bandage is to be applied, no matter what color the individual's skin is.

DE-U-8811658.1, upon which the preamble of claim 1 is based, discloses a device for covering skin blemishes, scars and wrinkles. The device takes the form of a thin material, such as transparent, flexible, soft skin-friendly and allergy-tested material, such as for example, plastic, rubber, special paper, silk or the like, and is provided on one side with a skin-friendly, self-adhesive layer and on the other side with a surface structure which is matched or similar to human skin. The device is designed in such a way that cosmetic or medicinal preparations adhere to it.

According to a first aspect of the present invention there is provided a bandage assembly for concealing a section of skin on a person, comprising:
(a) a segment of adhesive tape for attachment to (i) said section of skin and (ii) skin outwardly adjacent said section, said skin having a predetermined colouration, and said segment of tape having a top surface and a periphery; and
a layer of make-up;
said tape having a texture that retains the layer of make-up;
   characterised in that said layer of make-up has a colouration that matches the colouration of said skin, said layer covering said top surface and periphery of the tape and the skin outwardly adjacent said section of skin and said periphery, thereby concealing said section of skin and the segment of adhesive tape, said tape having a thinness that permits the assembly to blend with the skin at said periphery.

According to a second aspect of the present invention there is provided a method of concealing a section of skin on a person, the method comprising:
(a) affixing a segment of adhesive tape to said section of skin and to skin which is outwardly adjacent said section of skin, said segment having a top surface and a periphery; and
(b) covering the top surface and periphery of the segment of adhesive tape and the skin outwardly adjacent said section of skin and said periphery with a layer of make-up having a colouration that matches the colouration of the outwardly adjacent skin, said tape having a texture that retains the layer of make-up and a thinness that permits the make-up covered tape to blend with the skin at said periphery.

According to a third aspect of the present invention there is provided a kit for concealing a section of skin on a person, comprising a packaged combination:
(a) a container for containing a flowable make-up whose colouration matches the colouration of the section of skin; and
(b) a segment of adhesive tape that is sized and shaped to overlie said section, said tape having a top surface having a texture that allows the tape to retain said make-up when it is applied thereto and said tape having a thinness that permits the tape to blend with said section of skin when the tape is covered with a layer of the make-up.

Embodiments of device in accordance with the present invention will now be described, by way of example only, with reference to the following drawings (not to scale), in which:
Figure 1 is a sectional plan exploded view of a skin wound and a segment of adhesive tape adapted to cover the wound;
Figure 2 is a sectional plan view of the wound covered with the tape;
Figure 3 is a sectional plan schematic view showing make-up being applied to the top face surface of the tape;
Figure 4 is a sectional plan view of the wound covered with the tape and make-up;
Figures 5 and 6 are sectional plan views of an alternative embodiment of the bandage assembly shown in Figures 1-4.
Figure 7 is a sectional plan view of another embodiment of the invention.

In the drawings like parts or elements are referred to by the same reference numerals.

This invention provides for cosmetically acceptable wound/blemish covers which conform to skin contours and surfaces, are porous to promote healing and can, if medically appropriate, provide a therapeutic environment in which a wound can heal or a blemish, such as a pimple, can speedily recede. Such covers are herein termed "invisible bandages" wherein that term is defined within the context of this invention as bandage assemblies that are not readily apparent to an observer in that they blend with the skin of the person to whom they are applied and have a feathered edge that makes the transition between the assembly and the skin less discernible.

The invisible bandages match the skin of the person to whom they are applied in that the make-up is selected to match the skin appearance (color, texture, reflectivity) and applied to the bandage in such a manner as to camouflage the bandage. The matching make-up can be any type of make-up including liquid, cream or stick types, provided they have the flowability and malleability to be able to be spread to effectively cover the underlying adhesive tape. Ideally, the make-up is non-greasy, non-smearable, matches the appearance of the skin to which it is applied (i.e., closely resembles the skin in coloration, texture or finish, and reflectivity), is sterilizable, and retains its appearance on both skin and the underlying adhesive tape upon drying and aging. Opaque make-ups having such characteristics may be obtained from commercial sources or custom formulated. They will typically contain vehicles, solvents, emollients, pigments, surfactants, and reflective materials. Examples of commercially available make-ups are those manufactured by Revlon, Inc., Charles of the Ritz Group, Ltd., Noxell Corp., Estee Lauder, and Orlane. "Cover-up" cosmetics such as DERMABLEND™ cosmetics and COVERMARK™ cosmetics may also be used. As used herein, the term "match" is not limited to identity of appearance but intends to include make-ups that closely resemble the skin appearance.

There are many different embodiments for the invisible bandages of this invention. The invisible bandages can be made in any shape to conform to the contours of the surface and site to which they are to be applied. They are flexible, porous and have a low potential for sensitization. They can be modified to provide therapeutic environments for wound closure and healing.

However, for some uses wherein a wound, such as an abrasion, tissue rupture or surgical opening, is not present, thinness of the bandage assembly may be a very important criterion to maximize the cosmetic acceptability of the bandage. For example, it may be desired to conceal the presence of a scar, keloid or birthmark. For such uses among others, a representative invisible bandage of this invention comprises an adhesive tape, preferably a paper tape, more preferably a micropore-type of paper tape to which is applied a make-up that matches the skin tone of the person to whom the assembly is applied. It is preferred that the tape be applied first and that the make-up then be applied over the tape and blended around it such that both the underlying skin and the tape are concealed.

The tape used for the bandages of this invention can be any kind of tape that can be applied to skin without harm, for example, surgical tape. The tape for the invisible bandages of this invention are those tapes which are preferably flexible and have a texture that retains the cosmetic cover, preferably a paper tape. The paper tape is preferably a micropore-type of paper tape. Examples of paper types useful in this invention are SCANPOR™ surgical tape [distributed by Allerderm Labs, Mill valley, CA (U.S.A.)] and 3M 1529 paper tape. It has been found that the tackier the tape is, the better it is as a component of the invisible bandages of this invention.

Another preferred representative embodiment of the invisible bandages of this invention is one that comprises in addition to the tape, preferably paper tape, more preferably a micropore-type of paper tape to which the matching make-up is applied, a thin layer of a non-adhesive material that underlies a central portion of the paper tape, for example SUCH™ [Kendall Company, Boston, MA (U.S.A.)]. The non-adhesive layer prevents the tape from sticking to the surface of a wound, lesion, abrasion or dermatologic eruption, and thereby promotes the healing process by presenting a non-disruptive surface. It also makes the tape occlusive or semi-occlusive (i.e., it is permeable to gases, impermeable to pathogens, and has limited permeability to water vapor).

Another preferred representative embodiment of the invisible bandages of this invention comprises the adhesive tape, preferably paper tape, more preferably a micropore-type of paper tape to which the matching make-up is applied, a thin layer of non-adhesive material, and in addition, a middle layer between the tape and non-adhesive material, wherein said middle layer comprises an absorptive material and/or materials, such as GORTEX™ [W.L. Gore & Assoc, Elkton, MD (U.S.A.)], which are air permeable but permeable to liquids and vapors in varying degrees. The absorptive material, for example surgical gauze, gel or hydrocolloid, can be impregnated with medicaments, alone or in combination, that promote wound healing, for example, epidermal growth factors, steroids, antibiotics, hormones and other healing factors that promote therapeutic environments. Exemplary of such medicaments is a topical gel such as a formulation of Slindamycin phosphate, or a drying lotion such as that produced by Halina Andre Ltd. [Austin, Texas (U.S.A.)].

In certain types of wounds, a moist environment for healing is desirable. In such cases, the absorptive material in the middle layer of the invisible bandage may be air permeable but not particularly liquid permeable. Alternatively, the middle layer may comprise a material such as GORTEX™ which is permeable to air but only in varying degrees permeable to liquids and vapors and is impermeable to pathogens such as bacteria, either alone or in combination with an absorptive material.

Another representative embodiment of the invisible bandages of this invention would be that wherein the non-adhesive layer is not required or is not preferred. For example, a preferred environment for healing may require that an absorptive material impregnated with an appropriate medicant be directly in contact with the wound, abrasion or dermatologic eruption. One embodiment to meet that requirement would be that wherein the invisible bandage comprises tape, preferably paper tape, more preferably a micropore-type of paper tape to which the matching make-up is applied and absorptive material which is appropriately impregnated and upon application of the bandage would be in direct contact with the patient's skin. Another embodiment of this invention which would meet the same requirement is that wherein a material, such as GORTEX™, which is air permeable but permeable to liquids and vapors in varying degrees, is located in between the tape and an absorptive material layer that is impregnated with a liquid medicament. Thus, in that latter embodiment, the absorptive material would be directly in contact with the skin's surface supplying a therapeutic environment for whatever dermatologic condition is present which environment is further maintained by the presence of the air but variably liquid impermeable material just above the absorptive material.

A still further representative embodiment of this invention is that wherein the invisible bandage comprises tape, preferably paper tape, more preferably a micropore-type of paper tape to which the matching make-up is applied and a material, such as GORTEX™, which is air permeable but variably liquid impermeable. Such an embodiment would be preferred wherein a moist environment is desirable for the dermatologic condition, but topical applications are not necessary or are not preferred.

In another embodiment of the invisible bandages of this invention, medicaments, such as antibiotics, epidermal growth factors, drying lotions among others, are added to the matching make-up, preferably wherein the make-up is of a liquid type For example, in such an embodiment, the invisible bandage could comprise an appropriate type of tape and make-up to which has been added the appropriate medicament or medicaments. Another version of such an embodiment wherein the make-up contains a therapeutic agent is that which comprises an appropriate type of tape and a layer of absorptive material, such as surgical gauze, gel or hydrocolloid; whereas still another version would further compromise another thin layer of a non-adhesive material that would be in contact with the patient's skin. Of course, in such an embodiment wherein the make-up is liquid and contains one or more therapeutic agents, it would not be preferred for the invisible bandage to comprise a layer of material that is particularly liquid impermeable.

The drawings further illustrate specific embodiments of the bandage assembly of the invention.

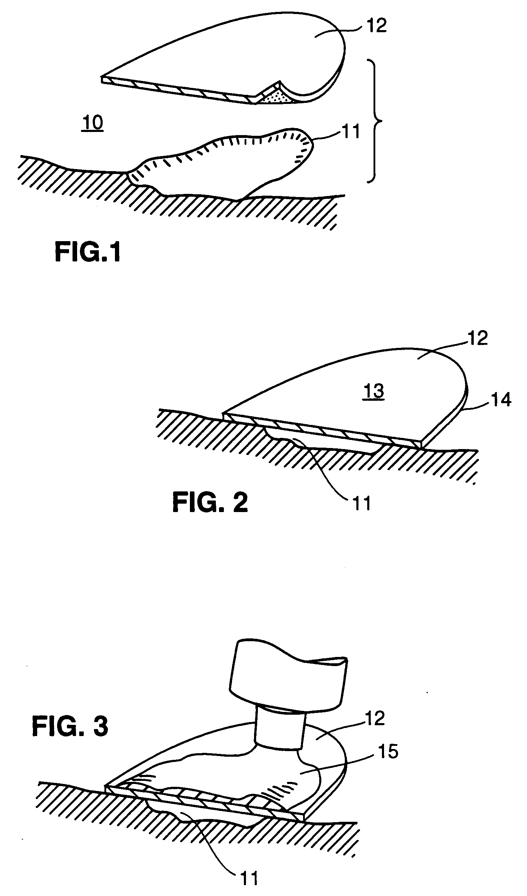

Figures 1-4 depict one embodiment of the bandage assembly of the invention and the procedure by which the assembly of the invention is applied to the skin.

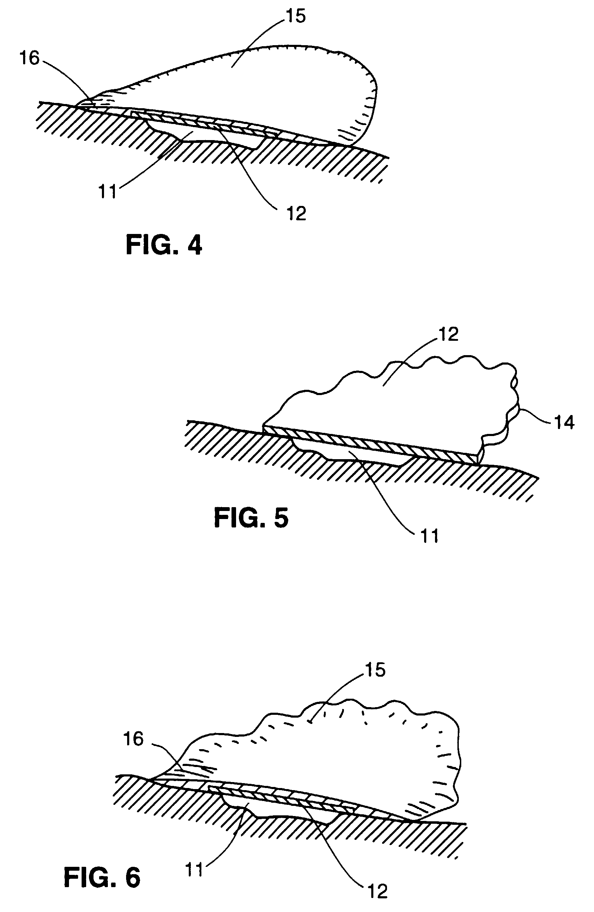

Figure 1 shows a section of human skin, designated 10, that has a wound 11 in it. A segment of adhesive tape 12 that is sized and shaped to overlie the wound is placed over the wound as shown in Figure 2. Such occlusion of the wound protects the wound and promotes healing. The segment has a top surface 13 and a periphery 14. A flowable or malleable make-up formulation, designated 15 in Figures 3 and 4, is applied to the top surface as shown in Figure 3 and then is spread to cover the periphery of the tape segment and the area of skin immediately surrounding the periphery. As shown in Figure 4 the make-up is smoothed and feathered in the area 16 outwardly of the periphery at a downward (toward the skin) angle from the periphery, so as to lessen the visibility of the transition, junction or edge between the make-up and the skin. As indicated, the appearance (coloration, texture, reflectivity) of the make-up is chosen to match the appearance of the skin. Such matching and the edge feathering of the make-up serve to conceal the assembly from casual observation.

Figures 5 and 6 depict a second embodiment of the assembly. This second embodiment is identical to the embodiment shown in Figures 1-4 except for the configuration of the periphery of the segment of the adhesive tape. Specifically, the embodiment of Figures 1-4 has a regular-shaped periphery (i.e., the segment has a smooth oval shape) whereas the periphery of segment of tape of the embodiment of Figures 5 and 6 has an irregular curvilinear shape. It is believed that an irregular-shaped assembly is even less discernible than a regular-shaped assembly and may adhere better to curved surfaces of the skin. Of course, it is within the scope of the invention to use a tape segment of virtually any shape.

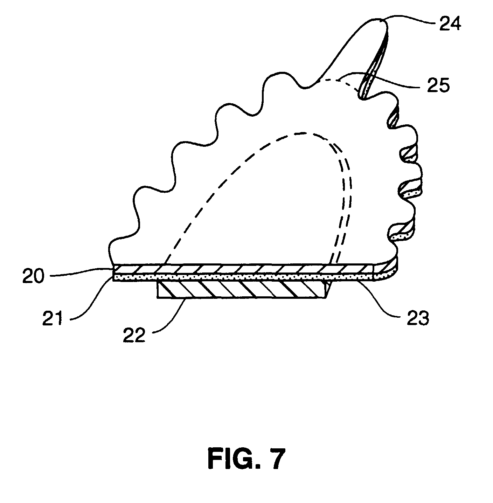

Figure 7 illustrates a preferred embodiment of a tape structure that is especially adapted to conceal a wound or blemish. The tape of Figure 7 is a three layer laminate composed of an upper paper backing layer 20, an underlying pressure-sensitive adhesive layer 21, and a central wound release layer 22 made of a material such as polyurethane which does not adhere to wounds or blemishes. As shown, layer 22 is not coterminous in size with layer 21 so that a peripheral ring 23 of exposed adhesive surrounds layer 22. This ring provides the means by which the assembly is affixed to the skin. As further shown, the assembly has a scalloped edge and the backing is provided with a removable tab, 24 that facilitates its removal from a conventional release liner layer (not shown). The backing is scored at 25 across the neck of the tab to facilitate removal of the tab from the remainder of the tape.

The invention further concerns methods of bandaging or dressing wounds in a cosmetically acceptable fashion and of concealing unsightly dermatologic conditions, such as birthmarks, scars, keloids, allergic reactions, varicose veins, bruises among others. An exemplary method of this invention comprises applying an appropriate segment of adhesive tape according to this invention as described above to conceal the dermatologic condition, and then applying make-up to the top surface and periphery of the tape and the immediately adjacent skin wherein said make-up matches the skin tone of the individual and is applied in such a manner as to conceal the periphery of the tape.

This invention still further provides for methods of non-surgically lifting facial or other bodily wrinkles and sags of the skin. A representative example of such a method comprises applying an appropriate tape according to this invention, preferably paper tape, more preferably a micropore-type of paper tape just next to the hairline of an individual who desires a non-surgical face-lift in such a manner as to pull up the skin below the bandage so that the skin appears more taut and smooth than it had appeared. The make-up that matches the skin tone of the person undergoing the non-surgical face-lift is applied in a manner that causes the bandage to blend with the person's complexion. The invisibility of the tape can be enhanced by appropriate hair styling.

The kits of the invention will comprise in packaged combination: (1) one or more segments of adhesive tape that are adapted to be affixed to human skin, and (2) a container of make-up having a predetermined coloration. The kits may contain additional containers of make-up of various coloration. The segments may be of various precut sizes and shapes or be of one size that may be cut to the desired size and shape. The kits may also contain instructions for applying the tape and make-up to the skin.

## Claims

1. A bandage assembly for concealing a section of skin on a person, comprising:
(a) a segment of adhesive tape (12) for attachment to (i) said section of skin and (ii) skin outwardly adjacent said section, said skin having a predetermined colouration, and said segment of tape having a top surface (13) and a periphery (14); and
a layer of make-up (15);
said tape (12) having a texture that retains the layer of make-up;
characterised in that said layer of make-up (15) has a colouration that matches the colouration of said skin, said layer covering said top surface (13) and periphery (14) of the tape and the skin outwardly adjacent said section of skin and said periphery, thereby concealing said section of skin and the segment of adhesive tape (12), said tape (12) having a thinness that permits the assembly to blend with the skin at said periphery.

2. A bandage assembly according to claim 1, wherein the make-up (15) is of a type selected from the group consisting of liquid, cream and stick types.

3. A bandage assembly according to claim 1 or claim 2, wherein said periphery has an irregular shape.

4. A bandage assembly according to any one of the preceding claims, wherein the portion of said layer of make-up (15) covering the skin outwardly adjacent said periphery (14) angles downward from said periphery.

5. A bandage assembly according to any one of the preceding claims, wherein the adhesive tape (12) is paper tape.

6. A bandage assembly according to claim 5, wherein the adhesive tape (12) is a micropore-type of paper tape.

7. A bandage assembly according to claim 6, wherein the micropore-type of paper tape (12) is a surgical tape which is tacky.

8. A bandage assembly according to any one of the preceding claims, further comprising a layer of material which is air permeable but variably liquid permeable which layer is in direct contact with the skin when the bandage is applied.

9. A bandage assembly according to any one of claims 1 to 7, further comprising a layer of absorptive material which is applied to the surface of a wound.

10. A bandage assembly according to claim 9, further comprising a layer of material which is air permeable but variably liquid permeable which is between the tape and the layer of absorptive material.

11. A bandage assembly according to any one of claims 1 to 7, wherein said section of skin defines a wound or blemish and the adhesive tape comprises a laminated composite of a backing layer (20), an underlying pressure-sensitive adhesive layer (21), and a basal layer of a wound or blemish release material (22), said basal layer being smaller in area than the adhesive layer so that the adhesive layer provides an exposed peripheral ring (23) about the basal layer.

12. A bandage assembly according to claim 11, further comprising a material which is air permeable but variably liquid permeable between the tape and the non-adhesive material.

13. A bandage assembly according to claim 11, further comprising an absorptive material between the tape and the wound or blemish release material.

14. A bandage assembly according to claim 13, wherein said absorptive material is surgical gauze.

15. A bandage assembly according to claim 13, further comprising in combination with said absorptive material a material which is air permeable but variably liquid permeable.

16. A bandage assembly according to any one of claims 13 to 15, wherein said absorptive material is impregnated with a therapeutic agent.

17. A bandage assembly according to claim 16 wherein said therapeutic agent is selected from the group consisting of antibiotics, drying lotions, epidermal growth factors, steroids, hormones, and healing factors.

18. A method of concealing a section of skin on a person, the method comprising:
(a) affixing a segment of adhesive tape (12,20) to said section of skin and to skin which is outwardly adjacent said section of skin, said segment having a top surface (13) and a periphery (14); and
(b) covering the top surface (13) and periphery (14) of the segment of adhesive tape (12,20) and the skin outwardly adjacent said section of skin and said periphery with a layer of make-up having a colouration that matches the colouration of the outwardly adjacent skin, said tape having a texture that retains the layer of make-up and a thinness that permits the make-up covered tape to blend with the skin at said periphery.

19. A kit for concealing a section of skin on a person, comprising a packaged combination:
(a) a container for containing a flowable make-up (15) whose colouration matches the colouration of the section of skin; and
(b) a segment of adhesive tape (12,20) that is sized and shaped to overlie said section, said tape having a top surface (13) having a texture that allows the tape to retain said make-up (15) when it is applied thereto and said tape having a thinness that permits the tape to blend with said section of skin when the tape is covered with a layer of the make-up.

## Patentansprüche

1. Verband zum Verbergen eines Abschnitts der Haut einer Person, umfassend:
a) ein Segment Klebeband (12) zum Anbringen an (i) dem Abschnitt der Haut und (ii) dem Abschnitt außen benachbarter Haut, wobei die Haut eine vorbestimmte Tönung besitzt und das Bandsegment eine Oberseite (13) und einen Umfang (14) aufweist; und
eine Make-Up-Schicht (15);
wobei das Band (12) eine Textur besitzt, welche die Make-Up-Schicht hält;
**dadurch gekennzeichnet**, daß die Make-Up-Schicht (15) eine Tönung aufweist, die angepaßt ist an die Tönung der Haut, wobei die Schicht die Oberseite (13) und den Umfang des Bandes und die außerhalb des Hautabschnitts und des Umfangs benachbarte Haut bedeckt, um dadurch den Hautabschnitt und das Segment des Klebebandes (12) zu verbergen, wobei das Band (12) eine Dicke besitzt, die es ermöglicht, daß der Verband sich mit der Haut an dem Umfang verwischt.

2. Verband nach Anspruch 1, bei dem das Make-Up (15) von einem Typ ist, der aus der Gruppe Flüssigkeit, Creme und Stift ausgewählt ist.

3. Verband nach Anspruch 1 oder 2, bei dem der Umfang eine unregelmäßige Form aufweist.

4. Verband nach einem der vorhergehenden Ansprüche, bei dem derjenige Bereich der Make-Up-Schicht (15), der die Haut in der äußeren Nachbarschaft des Umfangs (14) abdeckt, von dem Umfang nach unten abgewinkelt ist.

5. Verband nach einem der vorhergehenden Ansprüche, bei dem das Klebeband (12) Papierband ist.

6. Verband nach Anspruch 5, bei dem das Klebeband (12) ein Mikroporen-Papierband ist.

7. Verband nach Anspruch 6, bei dem das Mikroporen-Papierband (12) ein Klebriges chirurgisches Band ist.

8. Verband nach einem der vorhergehenden Ansprüche, weiterhin umfassend eine Schicht aus einem Material, welches luftdurchlässig, jedoch veränderlich flüssigkeitsdurchlässig ist, wobei diese Schicht sich in direkter Berührung mit der Haut befindet, wenn der Verband angelegt ist.

9. Verband nach einem der Ansprüche 1 bis 7, weiterhin umfassend eine Schicht aus absorbierendem Material, die auf die Oberfläche einer Wunde aufgebracht wird.

10. Verband nach Anspruch 9, weiterhin umfassend eine Schicht aus einem Material, das luftdurchlässig ist, jedoch veränderlich flüssigkeitsdurchlässig ist, wobei sich diese Schicht zwischen dem Band und der Schicht aus absorbierendem Material befindet.

11. Verband nach einem der Ansprüche 1 bis 7, bei dem der Abschnitt der Haut eine Wunde oder eine Verunstaltung ist, und das Klebeband ein Verbundlaminat aus einer Rückenschicht (20), einer darunterliegenden druckempfindlichen Klebstoffschicht (21) und einer Grundschicht aus einem Wunden- oder Verunstaltungs-Heilungsmaterial (22) ist, wobei die Grundschicht eine geringere Fläche hat als die Klebstoffschicht, so daß die Klebstoffschicht einen freiliegenden Umfangsring (23) um die Grundschicht herum bildet.

12. Verband nach Anspruch 11, umfassend ein Material, welches durchlässig, jedoch veränderlich flüssigkeitsdurchlässig ist und sich zwischen dem Band und dem klebstoffreien Material befindet.

13. Verband nach Anspruch 11, umfassend ein absorbierendes Material zwischen dem Band und dem Wunden- oder Verunstaltungs-Heilungsmaterial.

14. Verband nach Anspruch 13, bei dem das absorbierende Material chirurgische Gaze ist.

15. Verband nach Anspruch 13, der in Kombination mit dem absorbierenden Material ein Material aufweist, welches luftdurchlässig, jedoch veränderlich flüssigkeitsdurchlässig ist.

16. Verband nach einem der Ansprüche 13 bis 15, bei dem das absorbierende Material mit einem therapeutischen Mittel imprägniert ist.

17. Verband nach Anspruch 16, bei dem das therapeutische Mittel ausgewählt ist aus der Gruppe Antibiotika, Trocknungslotionen, Epidermiswachstumsfaktoren, Steroide, Hormone und Heilungsfaktoren.

18. Verfahren zum Verbergen eines Hautabschnitts einer Person, umfassend:
(a) Befestigen eines Klebebandsegments (12, 20) an dem Hautabschnitt und an der Haut, die dem Hautabschnitt außen benachbart ist, wobei das Segment eine Oberseite (13) und einen Umfang (14) besitzt;
(b) Bedecken der Oberseite (13) und des Umfangs (14) des Klebebandsegments (12, 14) und der dem Hautabschnitt und dem Umfang außen benachbarten Haut mit einer Schicht aus Make-Up mit einer Tönung, die an die Tönung der außen benachbarten Haut angepaßt ist, wobei das Band eine Textur besitzt, welche die Make-Up-Schicht hält, und eine Dicke aufweist, die es möglich macht, das mit Make-Up bedeckte Band mit der Haut an dem Umfang zu verwischen.

19. Pflegepack zum Verbergen eines Hautabschnitts einer Person, umfassend in verpackter Zusammenstellung:
a) einen Behälter zur Aufnahme eines fließfähigen Make-Ups (15), dessen Tönung an die Tönung des Hautabschnitts angepaßt ist; und
b) ein Klebebandsegment (12, 20), welches so bemessen und geformt ist, daß es über dem Abschnitt liegt, wobei das Band eine Oberseite (13) mit einer Textur aufweist, die es dem Band ermöglicht, das Make-Up (12) zu halten, wenn es darauf aufgebracht wird, und das Band eine Dicke besitzt, die es ihm ermöglicht, sich mit dem Hautabschnitt zu vermischen, wenn das Band mit einer Schicht aus dem Make-Up bedeckt ist.

## Revendications

1. Ensemble de pansement pour dissimuler une partie de la peau d'une personne, comportant :
(a) un troncon de bande adhésive (12) pour fixation sur (i) ladite partie de peau et (ii) sur la peau extérieurement adjacente a ladite partie, ladite peau ayant une coloration prédéterminée, et ledit tronçon de bande ayant une surface supérieure (13) et une périphérie (14), et
une couche de maquillage (15),
ladite bande (12) ayant une texture qui retient la couche de maquillage,
caractérisé en ce que ladite couche de maquillage (15) a une coloration qui correspond à la coloration de ladite peau, ladite couche recouvrant ladite surface supérieure (13) et ladite périphérie (14) de la bande et la peau extérieurement adjacente à ladite partie de peau et à ladite périphérie, de manière à dissimuler ladite partie de peau et le tronçon de bande adhésive (12), ladite bande (12) ayant une minceur qui permet a l'ensemble de se fondre à la peau à ladite périphérie.

2. Ensemble de pansement selon la revendication 1, dans lequel le maquillage (15) est d'un type sélectionné parmi le groupe constitué des types liquides, crèmes et bâtons.

3. Ensemble de pansement selon la revendication 1 ou 2, dans lequel ladite périphérie à une forme irrégulière.

4. Ensemble de pansement selon l'une quelconque des revendications précédentes, dans lequel la partie de ladite couche de maquillage (15) recouvrant la peau extérieurement adjacente à ladite périphérie (15) est inclinée vers le bas à partir de ladite périphérie.

5. Ensemble de pansement selon l'une quelconque des revendications précédentes, dans lequel la bande adhésive (12) est une bande de papier.

6. Ensemble de pansement selon la revendication 5, dans lequel la bande adhésive (12) est une bande de papier de type à micropores.

7. Ensemble de pansement selon la revendication 6, dans lequel ladite bande de papier de type à micropores (12) est une bande chirurgicale collante.

8. Ensemble de pansement selon l'un quelconque des revendications précédentes, comportant de plus une couche de matière qui est perméable à l'air mais perméable aux liquides de manière variable, laquelle couche est en contact direct avec la peau lorsque le pansement est appliqué.

9. Ensemble de pansement selon l'une quelconque des revendications 1 à 7, comportant de plus une couche de matière absorbante qui est appliquée sur la surface d'une blessure.

10. Ensemble de pansement selon la revendication 9, comportant de plus une couche de matière qui est perméable à l'air, mais perméable aux liquides de manière variable, qui est située entre la bande et la couche de matière absorbante.

11. Ensemble de pansement selon l'une quelconque des revendications l à 7, dans lequel ladite partie de peau définit une blessure ou une imperfection, et la bande adhésive comporte un composite stratifié constitué d'une couche de renfort (20), d'une couche sous-jacente d'adhésif sensible à la pression (21), et d'une couche de base constituée d'une matière anti-adhérente vis-à-vis de la blessure ou de l'imperfection (22), ladite couche de base ayant une surface plus petite que la couche adhésive de sorte que la couche adhésive fournit un anneau périphérique exposé (23) autour de la couche de base.

12. Ensemble de pansement selon la revendication 11, comportant de plus une matière qui est perméable à l'air mais perméable aux liquides de manière variable, située entre la bande et la matière non-adhésive.

13. Ensemble de pansement selon la revendication 11, comportant de plus une matière absorbante entre la bande et la matière anti-adhérente vis-à-vis de la blessure ou de l'imperfection.

14. Ensemble de pansement selon la revendication 13, dans lequel ladite matière absorbante est une gaze chirurgicale.

15. Ensemble de pansement selon la revendication 13, comportant de plus en combinaison avec ladite matière absorbante, une matière qui est perméable à l'air mais perméable aux liquides de manière variable.

16. Ensemble de pansement selon l'une quelconque des revendications 13 à 15 dans lequel ladite matière absorbante est imprégnée d'un agent thérapeutique.

17. Ensemble de pansement selon la revendication 16, dans lequel ledit agent thérapeutique est sélectionné parmi le groupe constitué d'antibiotiques, de lotions séchantes, de facteurs de croissance de l'épiderme, de stéroïdes, d'hormones, et de facteurs de guérison.

18. Procédé de dissimulation d'une partie de la peau d'une personne, ledit procédé comportant les étapes consistant à :
(a) fixer un tronçon de bande adhésive (12, 20) sur ladite partie de peau et sur la peau qui est extérieurement adjacente à ladite partie de peau, ledit tronçon ayant une surface supérieure (13) et une périphérie (14), et
(b) recouvrir la surface supérieure (13) et la périphérie (14) du tronçon de bande adhésive (12, 20) et la peau extérieurement adjacente à ladite partie de peau et à ladite périphérie, d'une couche de maquillage ayant une coloration qui correspond à la coloration de la peau extérieurement adjacente, ladite bande ayant une texture qui retient la couche de maquillage et une minceur qui permet à la bande recouverte de matière de maquillage de se fondre à la peau à ladite périphérie.

19. Kit de dissimulation d'une partie de la peau d'une personne comportant une combinaison emballée constituée de :
(a) un conteneur destiné à contenir un maquillage pouvant s'étaler (15) dont la coloration correspond à la coloration de la partie de peau, et
(b) un tronçon de bande adhésive (12, 20) qui est dimensionné et conformé pour recouvrir ladite partie, ladite bande ayant une surface supérieure (13) ayant une texture qui permet à la bande de retenir ledit maquillage (15) lorsqu'il est appliqué sur celle-ci, ladite bande ayant une minceur qui permet à la bande de se fondre à ladite partie de peau lorsque la bande est recouverte d'une couche du maquillage.
